# EUROPEAN PATENT SPECIFICATION

(11) **EP 0 650 706 B1**
(45) Date of publication and mention of the grant of the patent: **06.05.1998**
(21) Application number: 94307797.4
(22) Date of filing: 25.10.1994
(51) Int. Cl.: A61F 2/46

(54) **Apparatus for implanting an acetabular cup**
Vorrichtung zum Implantieren einer Hüftgelenkspfanne
Appareil d'implantation d'une cupule acétabulaire

(30) Priority: 29.10.1993 GB 9322383
(43) Date of publication of application: 03.05.1995
(73) Proprietor: HOWMEDICA INTERNATIONAL INC., Shannon Co. Clare (IE)
(72) Inventor: Ling, Robin Sydney Mackwood, Dartmouth, South Devon TQ6 0HB (GB); Lawes, Peter, Maidenhead, Berkshire SL6 4DB (GB)
(74) Representative: Bridge-Butler, Alan James

(56) References cited:
- EP-A- 0 073 604
- EP-A- 0 542 432
- WO-A-85/04567
- US-A- 5 098 437

## Description

This invention relates to apparatus for implanting an acetabular cup which is to be held in place by cement.

It is known, for example, from the Charnley (type) ogee Flanged Cup to provide a flange on the cup to pressurise the cement but this only occurs as the cup approaches its final position in the acetabulum. The flexible flange touches the acetabular margin ending to occlude cement flow.

Another device for pressuring cement is known as the Exeter Acetabular Pressuriser. In this arrangement a water filled seal mounted on the end of a handle is used after the acetabulum has been filled with cement but before the cup is introduced, the seal covers the acetabulum and the surgeon pushes on the handle thereby forcing the doughy cement in the holes, ridges and trabeculae. When the instrument is removed and pressure is released blood flow can squeeze between the cement and the bone.

It is also known to provide cup positioning instruments with long pointers (vertical, horizontal and at right angles to the patient's centre line of body symmetry) to enable the surgeon to control cup orientation reasonably well provided the pelvis does not move. Linear positioning of the cup is determined by eye in a medio-lateral and anterio-posterior sense. If the natural acetabulum is well formed a by-eye judgement can be acceptable but many acetabulae are deformed or eroded in which case by-eye judgement can be confused.

A further method is to use what are usually referred to as acetabular spacers. These can be lugs fixed to the outside of the acetabular cup or can be press studs pushed into the bone base of the natural acetabulum. These prevent the cup from being pushed too deeply into the acetabular socket and can control medio-lateral and anterio-posterior positioning. However, they force some degree of centralisation of the cup. If the surgeon chooses to place the cup eccentrically to make use of a particular area of solid bone support, acetabular spacers do not help.

WO-A-8504567 shows a construction in which a flexible plastic sleeve is attached to the bone around the acetabular recess so that when cement is deposited therein it does not spread to other bone surfaces. The sleeve is made from flexible material and its internal diameter is greater than the diameter of the cup to be inserted. However no guidance or control is provided and the cup can be inserted at any position or attitude in the cement.

The present invention is intended to provide apparatus for overcoming some of the difficulties referred to above and to allow pressurisation of the cement so that good holding is obtained.

According to the present invention apparatus for implanting an acetabular cup comprises a loading tube and a suitably dimensioned acetabular cup, the loading tube being adapted for alignment with an acetabular socket, the bore of said tube being dimensioned to receive and engage the outer periphery of said cup to guide it into the socket and an inserter for pushing said acetabular cup down said loading tube into the socket.

Preferably the inserter for pushing the cup down the tube acts on the upper rim of the cup. It can be in the form of a piston which is a sliding fit within the bore of the tube. Thus in this form it can be used to push the cement down the tube if it is applied in this manner and/or the inserter can be used to push the cup down the tube.

The apparatus can include a flexible skirt on said tube.

With this apparatus it is possible to either fill the acetabulum with doughy cement and then reposition the skirt and tube assembly or the tube on the bone using markers to ensure that a previously chosen position and orientation are re-established, or the cement can be injected, for example by a cement gun, through the tube. The surgeon can then push the cup into the cement to create a pressure and ensure that the cement fills all the openings in the socket, if the flexible skirt is used it can be secured around the rim of the socket preventing the escape of cement and allowing it to be pressurised.

The method employing the apparatus can include removing the tube but leaving the flexible skirt in place after the cup has been located, alternatively the skirt can be removed after location of the cup and setting of the cement.

The flexible skirt can be held in place by means of screws, pins and/or staples, or finger pressure alone.

The flexible skirt can be provided with a flow control orifice and thus the method of operation can include allowing some of the pressurised cement to escape from the socket and through the control orifice.

The skirt can be integral with the tube or it can be removable.

Preferably the flexible skirt is made from a synthetic plastics material and if it is to be left in place could be polyethylene, polypropylene, polyurethane, or even a resorbable material for example polyglycolic acid, or polylactic acid.

In order to locate the cup the surgeon can work to a measurement or mark on the tube. By controlling the release of cement through the orifice in the skirt, he can push the cup thereby feeling the desired level of cement pressure. If he is not able to reach the desired measurement or mark he can release the pressure on the cement, allowing a little more cement to escape whilst the cup progresses further down the inside of the tube.

If desired a projecting collar can be provided in the inside of the tube and which is dimensioned to engage a flange on a cup with which it is to be used to prevent the surgeon from pushing the cup in too far. When this arrangement is used it is desirable to leave a part of the flexible skirt implanted.

Means can be provided for adjusting the diameter of the tube.

An advantage of the invention is that no spacers are required which tend to cause discontinuities in the cement mantle and which in turn increases the risk of the cement cracking. The arrangement also enables the surgeon to accurately locate the position and attitude of the cup.

Means can also be included for positioning and orientating the tube or the tube and skirt on the bone into which the cup is to be implanted.

The invention can be performed in various ways and various embodiments will now be described by way of example and with reference to the accompanying drawings in which :
Figure 1 is a diagrammatic cross-sectional view of one example of the apparatus showing the method of operation;
Figure 2 is a diagrammatic side view of another form of the apparatus which would be supplied as a series of designs with a wedge or sloping orientation to accommodate deformed acetabular socket rims;
Figures 3, 4 and 5 show how a step can be machined around the rim of the acetabulum showing how the apparatus can be used if the bony rim is eroded;
Figure 6 shows in diagram form how an orientation guide can be provided;
Figures 7, 8 and 9 show three steps in performing the method and using the apparatus when using a trial cup; and,
Figure 10 shows a method of using a guide tube without a skirt.

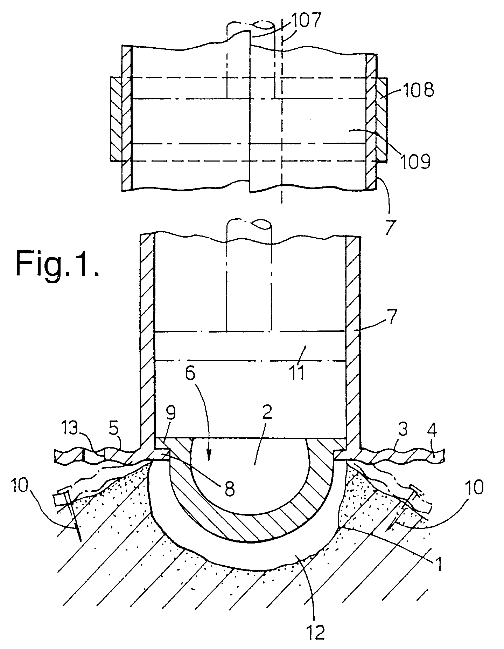

Figure 1 shows a general form of the apparatus, an acetabular socket being indicated by reference numeral 1 into which an acetabular cup 2 is to be fitted.

In order to fit the cup into the socket 1 in which it is to be held by cement a flexible skirt assembly 3 is utilised. This skirt assembly 3 is made as an integral unit from a synthetic plastics material, for example polyethylene, polypropylene, polyurethane, or even a resorbable material, for example polyglycolic acid, or polylactic acid. The assembly comprises a flexible skirt 4 which is shown with corrugations 5. The skirt has a central opening 6 and is provided with a loading tube 7 the inner bore of which is substantially the same diameter as the opening 6. The lower end of the loading tube is formed with a projecting collar 8 which is dimensioned to engage a projecting flange 9 provided on the cup 2.

To implant the cup 2 the acetabular socket 1 is first prepared in the usual way. The flexible skirt assembly 3 is now placed in he position shown in full lines in the drawing and the skirt 4 is bent and fixed to the bone around the acetabular opening by pins, screws or staples indicated by reference numeral 10. The deformed position of the skirt when in its secured position is indicated in broken lines.

If desired the skirt 4 can be preformed to the required shape. The relative positioning on to the acetabular bone can be controlled by positioning guides, with or without fixing.

Prior to fastening it in position however the position and orientation of the tube 7 is determined during the early preparatory stages of the operation and once this has been determined the skirt can be trimmed if necessary and fastened in position. It will be seen that on the right hand side of the drawing the width of the skirt has been trimmed down to assist in location.

The prepared acetabulum is now filled with cement through the tube 7, the cement being pushed into place by a manually operated inserter in the form of a piston 11 which is a sliding fit in the tube 7. The piston is removed when the cement is partially set and in an adequately doughy stage. The cup 2 is immediately inserted and passed down the piston tube 7 by the same or a different inserter and thus the cement is pressurised from the moment the cup touches it. The co-operating collar 8 and flange 9 of the cup engage thereby preventing the surgeon from pushing the cup in too far.

As will be seen from the drawing no spacers are required in the cement mantle which now surrounds the cup and which is indicated by reference numeral 12.

A cement flow release arrangement is provided by the provision of an orifice 13 in the skirt 4. The whole in the skirt is occluded, for example by the surgeon's thumb, to thus allow the pressure to build up and then allow excess cement to escape.

With the cup 2 in place and the cement fully cured the tube is cut away and the remainder of the skirt left implanted.

In the arrangement described above the tube is integral with the skirt 4 but if desired it could be made as a separate detachable item. Again, if desired and if no flange 9 on the cup is utilised it is possible to completely remove the flexible skirt 4 after the cup has been inserted, the skirt, nevertheless, providing the facility for pressuring the cement during insertion.

The use of the skirt 4 also enables the position and orientation of the cup to be determined prior to actual insertion and to allow the surgeon to place it precisely where he wants it in the acetabular socket.

It will of course be necessary to provide skirts in a choice of internal shapes and sizes to suit the chosen cup shape and size. the tube can also be provided in a similar choice of diameters but an adjustable tube could be set to the required diameter and fixed with a circumferential strap, an inserter for example in the form of a piston of appropriate dimensions being employed. Such a construction is shown at the upper end of the tube in Figure 1. The tube is split and overlaps at its edges as indicated by reference numeral 107 and is held at the required diameter by a strap 108, a larger size piston 109 is shown.

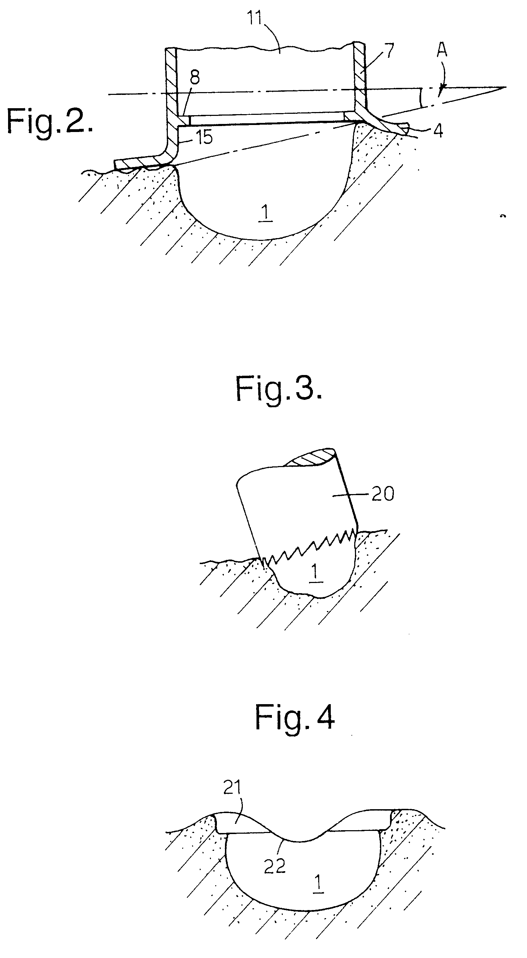

Figure 2 shows a construction of combined skirt 4 and tube 7 which can be used for deformed/eroded acetabular rims. The tubes and skirts can be supplied as a series of designs with a wedge or slope orientation to accommodate different angles of deformity, the slope angle in Figure 2 is indicated by reference numeral "A".

As will be seen in Figure 2 in this construction a projecting collar 8 is maintained substantially normal to the inner bore of the tube 7 but the length of inner bore wall 15 below the projecting collar 8 varies about the diameter to provide the angled effect shown.

The series of designs could of course also be provided in different sizes as well as in wedge/ramp angles.

This construction can also be provided by arranging for the skirt 4 and lower part of the tube wall 15 to be deformable so that the desired shape and wedge/ramp angle is formed by the operating surgeon.

The natural bony rim of the acetabulum is not a uniplanar circular feature and if desired the surgeon can machine a step around the rim to receive a sealing feature or the like.

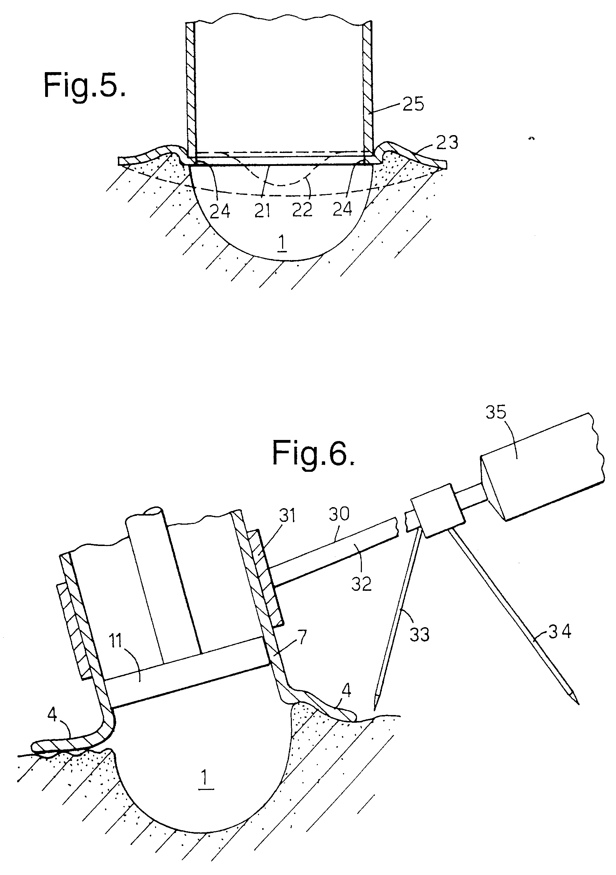

Figures 3 and 4 shows how a trepanning cutter 20 is used to machine the rim depth to provide a step 21 inside the natural bony rim. The natural depression in the rim of the acetabulum is indicated by reference numeral 22. Figure 5 shows how a skirt 23 can be used which provides an inwardly directed ledge 24 when placed on the step 21. The skirt extends upwardly and then outwardly and the removable tube 25 is placed in position on it. As will be seen from Figure 5 the deformable skirt 23 is large enough to extend around and cover the natural dip and prevent loss of cement when the piston 11 (not shown in Figure 5) is operated.

The ledge 24 in this construction can act in a similar manner to the projecting collar shown in Figure 2.

The loading tube 25 can be arranged so that it is a relative push fit into the walls of the step 21 where they are covered by the upstanding portion of the skirt 23.

It will be appreciated that a tube 25 fixed to the skirts 23 could be used if desired.

In present surgical techniques positioning and orientating an acetabular cup is sometimes achieved using a cup introducer/inserter with two alignment pointers, one of which points at 90° across the centre line (axis of symmetry) of the patient and the other points vertically. This controls only orientation. Position and depth of insertion are chosen by the surgeon by eye.

In the construction shown in Figure 6 the same reference numerals are used to indicate similar parts as shown in Figures 1 and 2 and an orientation guide 30 is provided which has a carrier 31 which surrounds the tube 7 and a long handle 32. The handle 32 carries adjustable alignment pointers 33, 34.

The flexible skirt 4 can be steered by the handheld alignment and orientation guide 30 which is provided with a handle grip 35. The skirt is flexible enough to cope with deformed bony rims around the natural acetabulum and the alignment and orientation guide keeps everything in position and maintains the skirt 4 tight against the bone.

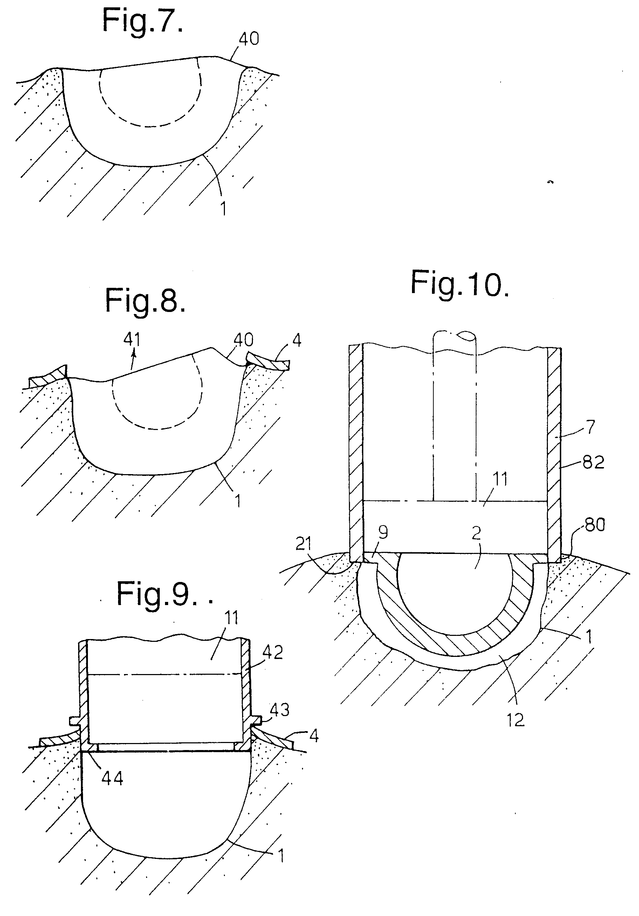

Figures 7 and 8 show an alternative method of employing the apparatus. An important task in this type of operation is to define the desired position and orientation of the cup. This is usually done by putting a trial or dummy implant in place. Theoretically it could be determined before the operation using X-rays.

When using the present invention it is necessary to place the tube and skirt such that the cup will eventually be positioned and tilted as required. The tube and skirt might be placed over a trial/dummy cup fixed and then the trial/dummy removed. Alternatively the surgeon might remove the trial/dummy first and immediately fit the tube and skirt on the basis that his by-eye judgement of position and orientation is good enough.

Figure 7 shows a prepared acetabulum 1 into which a trial acetabular cup 40 has been fitted. At this stage the surgeon chooses the position of centre and orientation.

Figure 8 shows how the surgeon can now fit the flexible skirt 4 around the trial cup 40 and the trial cup can then be removed, as indicated by arrow 41, without disturbing the skirt 4.

After the trial cup 40 has been removed, leaving the skirt in place, there is space for the tube (with or without an internally projecting collar) to be assembled to the skirt, for example as shown in Figure 9, and for the implant equivalent to the trial cup 40 but necessarily smaller in size to be put in position.

Figure 9 shows one way of carrying out the procedure referred to above by using a separable tube 42 which is pushed into place within the opening in the skirt 4, the flexible construction of the skirt enabling the tube to be pushed into place with a tight sliding fit. If desired a depth stop abutment ring 43 can be provided to control the insertion of the tube into the skirt, as shown in Figure 9. Due to the deformities of the natural acetabulum this abutment ring 43 will not necessarily contact the skirt around the full periphery. If desired the abutment ring 43 could be a tight sliding fit on the tube 42 so that it could be moved under pressure on the tube to provide an adjustable depth stop.

The lower end of the tube can have an optional collar 44 to act as a stop for the acetabular cup, with this arrangement the tube can be cut away once the cup has been inserted. If there is no such collar then the tube can be simply dismantled from the cup and skirt.

In figure 10 another method of implanting the cup 2 is shown and the same reference numerals as those used in Figure 1 are again used to indicate similar parts. With this method no skirt 3 is used.

The surgeon first prepares the socket 12 in the usual way and then employs a trepanning cutter in the manner shown in figures 3 and 4 to provide a step 21 inside the natural bony rim. The outer wall 80 of the step 2 is dimensioned so that the outer wall 82 of the tube 7 is a push fit onto the step 21.

In order to implant the cup the surgeon first places the tube 7 in position on the step 21 and the cement is then pushed into place by manual operation of the piston 11. The piston is removed when the cement is partially set and in an adequately doughy stage. The cup is now immediately inserted down the tube 7 and the cement is pressurised from the moment the cup touches it. The cup is pushed further downwards into the socket 12 to the desired position. A collar can be provided on the tube 7 as described in the construction shown in Figure 1, or a flange can be used.

An adjustable tube 7 can be used, for example, of the kind shown in Figure 1.

When an inserter is only used for pushing the cup down the tube it need not be in the form of a piston but merely comprise a suitable elongate rod or similar device to provide the pushing effect from the outer end of the tube.

Once again it will be seen from the drawing that no spacers are required in the cement mantle 12.

It will be appreciated that variations of the various embodiments described can be made by incorporating the different details described together.

The essence of the present invention is to provide continuous cement pressurisation during the fitting of the socket.

## Claims

1. Apparatus for implanting an acetabular cup comprising a loading tube (7) and a suitably dimensioned acetabular cup (2), the loading tube (7) being adapted for alignment with an acetabular socket (1), the bore of said tube (7) being dimensioned to receive and engage the outer periphery of said cup (2) to guide it into the socket (1) and an inserter (11) for pushing said acetabular cup (2) down said loading tube (7) into the socket (1).

2. Apparatus as claimed in claim 1 characterised in that said inserter (11) acts on the upper rim of the cup (2).

3. Apparatus as claimed in claim 2 characterised in that said inserter (11) is in the form of a piston which is a sliding fit within the bore of the tube (7).

4. Apparatus as claimed in any one of claims 1 to 3 characterised in that the bore of the tube (7) is provided with a projecting collar (8) dimensioned to engage the cup (2) with which it is to be used.

5. Apparatus as claimed in any one of the preceding claims 1 to 4 characterised in that said tube (7) is provided with a flexible skirt (4).

6. Apparatus as claimed in claim 5 characterised in that said skirt (4) is detachable from said tube (7).

7. Apparatus as claimed in claim 5 characterised in that said skirt (4) is integral with said tube (7).

8. Apparatus as claimed in any one of preceding claims 5, 6 or 7 characterised in that said skirt (4) is corrugated (5).

9. Apparatus as claimed in any one of preceding claims 5, 6, 7 or 8 characterised in that a flow control orifice (13) is provided in said skirt (4).

10. Apparatus as claimed in any one of preceding claims characterised in that one end of said tube (7) has a wedge or slope shaped orientation (A).

11. Apparatus as claimed in any one of the preceding claims characterised in that one end of the tube (7) is deformable.

12. Apparatus as claimed in any one of the preceding claims characterised by means (30) for orientating the tube (7) or tube (7) and/or skirt (4) on the bone into which the cup (2) is to be inserted.

13. Apparatus as claimed in any one of the preceding claims characterised in that said tube (7) and/or skirt (4) are made from a synthetic plastics material.

14. Apparatus as claimed in claim 13 characterised in that said skirt (4) is made from polyethylene, polypropylene or polyurethane.

15. Apparatus as claimed in any one of preceding claims 5 to 12 characterised in that said skirt (4) is made from a resorbable material.

16. Apparatus as claimed in claim 15 characterised in that said skirt (4) is made from polyglycolic acid or polylactic acid.

17. Apparatus as claimed in any one of the preceding claims characterised in that said tube (7) carries a measurement or mark to assist in locating said cup (2).

18. Apparatus as claimed in any one of the preceding claims characterised in that means (107,108) are provided for adjusting the diameter of said tube (7).

## Patentansprüche

1. Vorrichtung zum Implantieren einer Hüftgelenkpfanne, umfassend ein Beschickungsrohr (7) und eine geeignet bemessene Hüftgelenkpfanne (2), wobei das Beschickungsrohr (7) zum Ausrichten auf eine Hüftgelenkpfannenhöhle (1) ausgelegt ist und die Bohrung des Rohrs (7) zum Aufnehmen und in Eingriff treten mit der äußeren Peripherie der Hüftgelenkpfanne (2) bemessen ist, um diese in die Höhle (1) zu leiten, und eine Einführvorrichtung (11) zum Hinunterdrücken der Hüftgelenkpfanne (2) durch das Beschickungsrohr (7) in die Höhle (1).

2. Vorrichtung nach Anspruch 1, dadurch gekennzeichnet, daß die Einführvorrichtung (11) auf den oberen Rand der Pfanne (2) wirkt.

3. Vorrichtung nach Anspruch 2, dadurch gekennzeichnet, daß die Einführvorrichtung (11) in Form eines Kolbens vorliegt, der gleitend in die Bohrung des Rohrs (7) paßt.

4. Vorrichtung nach einem der Ansprüche 1 bis 3, dadurch gekennzeichnet, daß die Bohrung des Rohrs (7) mit einem vorstehenden Kragen versehen ist, der so bemessen ist, daß er mit der Pfanne in Eingriff tritt, mit der er verwendet werden soll.

5. Vorrichtung nach einem der vorstehenden Ansprüche 1 bis 4, dadurch gekennzeichnet, daß das Rohr (7) mit einer flexiblen Schürze (4) ausgestattet ist.

6. Vorrichtung nach Anspruch 5, dadurch gekennzeichnet, daß das die Schürze (4) vom Rohr (7) abnehmbar ist.

7. Vorrichtung nach Anspruch 5, dadurch gekennzeichnet, daß die Schürze (4) und das Rohr (7) eine Einheit bilden.

8. Vorrichtung nach einem der vorstehenden Ansprüche 5, 6 oder 7, dadurch gekennzeichnet, daß die Schürze (4) gewellt ist (5).

9. Vorrichtung nach einem der vorstehenden Ansprüche 5, 6, 7 oder 8, dadurch gekennzeichnet, daß eine Flußsteueröffnung (13) in der Schürze (4) vorgesehen ist.

10. Vorrichtung nach einem der vorstehenden Ansprüche, dadurch gekennzeichnet, daß ein Ende des Rohrs (7) eine keil- oder schrägförmige Orientierung (A) aufweist.

11. Vorrichtung nach einem der vorstehenden Ansprüche, dadurch gekennzeichnet, daß ein Ende des Rohrs (7) verformbar ist.

12. Vorrichtung nach einem der vorstehenden Ansprüche, gekennzeichnet durch eine Einrichtung (30), zum Ausrichten des Rohrs (7) oder des Rohrs (7) und/oder der Schürze (4) auf den Knochen, in den die Pfanne (2) eingesetzt werden soll.

13. Vorrichtung nach einem der vorstehenden Ansprüche, dadurch gekennzeichnet, daß das Rohr (7) und/oder die Schürze (4) aus einem synthetischen Kunststoffmaterial hergestellt sind.

14. Vorrichtung nach Anspruch 13, dadurch gekennzeichnet, daß die Schürze (4) aus Polyethylen, Polypropylen oder Polyurethan hergestellt ist.

15. Vorrichtung nach einem der vorstehenden Ansprüche 5 bis 12, dadurch gekennzeichnet, daß die Schürze (4) aus einem resorbierbaren Material hergestellt ist.

16. Vorrichtung nach Anspruch 15, dadurch gekennzeichnet, daß die Schürze (4) aus Polyglykolsäure oder Polymilchsäure hergestellt ist.

17. Vorrichtung nach einem der vorstehenden Ansprüche, dadurch gekennzeichnet, daß das Rohr (7) eine Abmessung oder Markierung zum leichteren lokalisieren der Pfanne (2) trägt.

18. Vorrichtung nach einem der vorstehenden Ansprüche, dadurch gekennzeichnet, daß Einrichtungen (107, 108) zum Einstellen des Durchmessers des Rohrs (7) vorgesehen sind.

## Revendications

1. Dispositif pour implanter une cupule acétabulaire comportant un tube de chargement (7) et une cupule acétabulaire (2) dimensionnée de manière adaptée, le tube de chargement (7) étant adapté pour être aligné avec une alvéole acétabulaire (1), l'alésage dudit tube (7) étant dimensionné pour recevoir la périphérie extérieure de ladite cupule (2) et venir en prise avec celle-ci pour la guider dans l'alvéole (1) et un organe d'insertion (11) destiné à pousser ladite cupule acétabulaire (2) vers le bas dudit tube de chargement (7) jusque dans l'alvéole (1).

2. Dispositif selon la revendication 1, caractérisé en ce que ledit organe d'insertion (11) agit sur le rebord supérieur de la cupule (2).

3. Dispositif selon la revendication 2, caractérisé en ce que ledit organe d'insertion (11) a la forme d'un piston monté de manière coulissante dans l'alésage du tube (7).

4. Dispositif selon l'une quelconque des revendications 1 à 3, caractérisé en ce que l'alésage du tube (7) est muni d'une collerette en saillie (8) dimensionnée pour venir en contact avec la cupule (2) avec laquelle elle doit être utilisée.

5. Dispositif selon l'une quelconque des revendications 1 à 4, caractérisé en ce que ledit tube (7) est muni d'une jupe souple (4).

6. Dispositif selon la revendication 5, caractérisé en ce que ladite jupe (4) est détachable dudit tube (7).

7. Dispositif selon la revendication 5, caractérisé en ce que ladite jupe (4) est venue de matière avec ledit tube (7).

8. Dispositif selon l'une quelconque des revendications 5, 6 ou 7, caractérisé en ce que ladite jupe (4) est ondulée (5).

9. Dispositif selon l'une quelconque des revendications 5, 6, 7 ou 8, caractérisé en ce qu'un orifice de commande d'écoulement (13) est agencé dans ladite jupe (4).

10. Dispositif selon l'une quelconque des revendications précédentes, caractérisé en ce qu'une première extrémité dudit tube (7) a une orientation inclinée ou en forme de coin (A).

11. Dispositif selon l'une quelconque des revendications précédentes, caractérisé en ce qu'une première extrémité du tube (7) est déformable.

12. Dispositif selon l'une quelconque des revendications précédentes, caractérisé en ce qu'il comporte des moyens (30) destinés à orienter le tube (7) ou le tube (7) et/ou la jupe (4) sur l'os dans lequel doit être insérée la cupule (2).

13. Dispositif selon l'une quelconque des revendications précédentes, caractérisé en ce que ledit tube (7) et/ou la jupe (4) sont constitués d'une matière plastique synthétique.

14. Dispositif selon la revendication 13, caractérisé en ce que ladite jupe (4) est constituée de polyéthylène, de polypropylène ou de polyuréthanne.

15. Dispositif selon l'une quelconque des revendications 5 à 12, caractérisé en ce que ladite jupe (4) est constituée d'un matériau pouvant être résorbé.

16. Dispositif selon la revendication 15, caractérisé en ce que ladite jupe (4) est constituée d'acide polyglycolique ou d'acide polylactique.

17. Dispositif selon l'une quelconque des revendications précédentes, caractérisé en ce que ledit tube (7) supporte des mensurations ou un repère pour aider au positionnement de ladite cupule (2).

18. Dispositif selon l'une quelconque des revendications précédentes, caractérisé en ce que des moyens (107, 108) sont prévus pour régler le diamètre dudit tube (7).
